# EUROPÄISCHE PATENTSCHRIFT

(11) **EP 1 013 262 B1**
(45) Veröffentlichungstag und Bekanntmachung des Hinweises auf die Patenterteilung: **29.11.2006**
(21) Anmeldenummer: 99124015.1
(22) Anmeldetag: 08.12.1999
(51) Int. Cl.: A61Q 17/04, A61K 8/04, A61K 8/19, A61K 8/49, A61K 8/35

(54) **Kosmetische und dermatologische O/W-Emulsionen mit einem Gehalt an Bornitrid**
Cosmetic and dermatologic o/w-emulsions containing boron nitride
Emulsions H/E cosmétiques et dermatologiques contenant du nitrure de bore

(30) Priorität: 24.12.1998 DE 19860267
(43) Veröffentlichungstag der Anmeldung: 28.06.2000
(73) Patentinhaber: Beiersdorf Aktiengesellschaft, 20245 Hamburg (DE)
(72) Erfinder: Gers-Barlag, Heinrich, Dr., 25495 Kummerfeld (DE); Müller, Anja, 23843 Rümpel (DE); Kröpke, Rainer, 22869 Schenefeld (DE); Pape, Wolfgang, Dr., 22041 Hamburg (DE)

(56) Entgegenhaltungen:
- EP-A- 0 987 008
- EP-A- 1 055 410
- WO-A1-00/07549
- DE-A- 19 923 667
- FR-A- 2 746 301

## Beschreibung

Die vorliegende Erfindung betrifft kosmetische und dermatologische O/W-Emulsionen mit einem Gehalt an Bornitrid. In einer vorteilhaften Ausführungsform betrifft die vorliegende Erfindung eine Anwendung, welche es erlaubt, die Stabilität von O/W-Emulsionen zu steigern.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion ( W/O Emulsion, z. B. Butter) handelt es sich um das umgekehrte Prinzip, wobei der Grundcharakter hier durch das Öl bestimmt wird.

Kosmetische Emulsionen bestehen aus mindestens einer Fettphase (beispielsweise natürliche und mineralische Öle, Fettalkohole, Fettsäureester usw.) und mindestens einer Wasserphase (z. B. Wasser, Glycerin, Sorbit, Glykole usw.), die mit Hilfe von Emulgatoren fein ineinander verteilt werden. Um eine dauerhafte Dispergierung zu erreichen, ist der Zusatz einer grenzflächenaktiven Substanz notwendig. Sie bildet an der Phasengrenze Öl/Wasser Grenzflächenfilme aus, wodurch dem irreversiblen Zusammenfließen der Tröpfchen entgegengewirkt wird. Zur Stabilisierung von Emulsionen werden häufig auch Emulgatorgemische verwendet.

Vom thermodynamischen Standpunkt aus betrachtet sind Emulsionen grundsätzlich instabile Systeme. Dennoch gelingt es, kosmetische Emulsionen von jahrelanger Stabilität herzustellen. Eine Instabilität der Emulsion äußert sich im Brechen oder Aufrahmen der Emulsion, d. h. in der Ausbildung zweier oder mehrerer Schichten, in die die Wasser- und Fettphase unter Einschluß des jeweiligen Emulgators separieren. Eine Emulsion wird dann instabil, wenn sich die feindispergierten Teilchen wieder zu größeren Aggregaten zusammenballen und die sich berührenden Tröpfchen zusammenfließen. Dieser Vorgang wird auch als Koaleszenz bezeichnet. Dies führt bei einer O/W-Emulsion letzlich zu einer auf der wäßrigen Phase schwimmenden Ölschicht (Aufrahmung bzw. Ausölungen).

Der Stand der Technik kennt mehrere wesentliche Faktoren, die einen positiven Einfluß auf die Stabilität von O/W-Emulsionen haben:
1. feinste Verteilung der Phasen ineinander:
   Je feiner die eine Phase in der anderen verteilt ist, je kleiner damit die dispergierten Teilchen sind, umso stabiler ist die Emulsion.
2. möglichst geringer Unterschied in der Dichte der beiden Phasen,
3. ein ausgewogenes Phasenvolumenverhältnis.

Einfache Emulsionen lassen sich mit modernen Emulgatoren durchaus stabil formulieren. Sind aber größere Mengen schwierig zu emulgierender Fette und Öle, lösungsmittelhaltige Wirkstoffe, Pigmente oder ähnliche unlösliche Zusätze Bestandteil der Emulsion, dann sind Stabilisatoren nahezu unumgänglich. Dies gilt insbesondere für W/O-Emulsionen, aber auch O/W-Emulsionen können in ihrer Stabilität wirksam verbessert werden.

Disperse Zubereitungen vom Typ der Emulsionen spielen in den verschiedensten Bereichen der Kosmetikindustrie eine herausragende Rolle. So handelt es sich beispielsweise bei Hautpflegemitteln und Dermatika überwiegend um komplexe emulsionsförmige Präparate, deren Formulierung, Herstellung und Prüfung umfassende Kenntnisse verlangen.

Aus der FR 2 746 301 A sind stabile O/ W-Emulsionen enthaltend Bornitrid und Nanopigmente bekannt.

Eine Aufgabe der vorliegenden Erfindung war es also, kosmetische oder dermatologische O/W-Emulsionen zur Verfügung zu stellen, welche einfach zu formulieren sind und dabei eine hohe Stabilität aufweisen.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letzlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, Lipide, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Während die sogenannte UV-C-Strahlung mit einer Wellenlänge, die kleiner als 290 nm ist, keine physiologische Bedeutung hat, verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben. UV-A-Strahlung ist im Hinblick auf die Auslösung photodynamischer, speziell phototoxischer Reaktionen und chronischer Veränderungen der Haut noch weitaus gefährlicher als UV-B-Strahlung. Auch kann der schädigende Einfluß der UV-B-Strahlung durch UV-A-Strahlung zusätzlich verstärkt werden.

Zum Schutz gegen UV-Strahlung sind zahlreiche Verbindungen (UV-Filtersubstanzen) bekannt, bei denen es sich zumeist um polare Verbindungen handelt.

Kosmetische Lichtschutzmittel sollen, in dünner Schicht auf die Haut aufgetragen, diese vor den negativen Auswirkungen der Sonnenstrahlen schützen. UV-Filtersubstanzen absorbieren unerwünschte UV-Strahlung bzw. Teile der UV-Strahlung in der Schutzschicht des Sonnenschutzmittels auf der Haut.

Allerdings ist die Stabilität von O/W-Emulsionen mit polaren UV-Filtersubstanzen bzw. mit polaren Bestandteilen, welche zur Lösung von als Feststoff vorliegenden UV-Filtersubstanzen beitragen sollen, ohne den Zusatz unpolarer Öle im allgemeinen unzureichend. Phasentrennungen und Ausölungen erfolgen bei den Zubereitungen des Standes der Technik häufig bereits nach einer sehr kurzen Lagerzeit. Eine hohe Polarität der Ölphase ist aber bei Sonnenschutzformulierungen durchaus erwünscht, da ansonsten die (polaren) UV-Filtersubstanzen auskristallisieren könnten und ein ausreichender Lichtschutzfaktor dann nicht erreicht wird.

Eine weitere Aufgabe der vorliegenden Erfindung war es daher, den Nachteilen des Standes der Technik abzuhelfen und O/W-Emulsionen zur Verfügung zu stellen, welche auch bei einem hohen Gehalt an polaren Bestandteilen über einen langen Zeitraum lagerfähig und stabil sind.

Ferner war es Aufgabe der Erfindung, kosmetische und dermatologische Grundlagen für kosmetische und dermatologische Zubereitungen zur Verfügung gestellt werden, die sich durch gute Hautverträglichkeit auszeichnen.

Es war indes überraschend und für den Fachmann in keiner Weise vorauszusehen, daß kosmetische oder dermatologische O/W-Emulsionen gemäß Anspruch 1 den Nachteilen des Standes der Technik abhelfen würden.

Polare Ölkomponenten sind im Sinne der vorliegeden Erfindung Ölkomponenten, deren Grenzflächenspannung gegen Wasser (bestimmbar mit einem Ringtensiometer der Fa. Krüss, Ringtensiometer K10) kleiner als 35 mN/m (Milli-Newton pro Meter) ist. Unpolare Ölkomponenten sind dementsprechend solche, deren Grenzflächenspannung gegen Wasser (bestimmbar mit einem Ringtensiometer der Fa. Krüss, Ringtensiometer K10) größer als 35 mN/m ist.

Es war insbesondere für den Fachmann nicht vorauszusehen gewesen, daß erfindungsgemäße Zubereitungen auch ohne den Zusatz von unpolaren Ölen bei einem entsprechend hohen Gehalt an UV-Filtern (wie z.□B. symmetrisch oder unsymmetrisch substituierte Triazinderivate und/oder Benzotriazole) und/oder polaren Ölkomponenten eine hervorragende Stabilität gegenüber Zerfall in Öl- und Wasserphasen aufweisen würden.

Erfindungsgemäß ist daher weiterhin auch die Verwendung nach einem der Ansprüche 2 und 3.

Bornitrid (BN) existiert in drei natürlichen Modifikationen, nämlich als graphitanaloges hexagonales α-BN, als diamantanaloges kubisches β-BN (Borazon) ud als metastabiles γ-BN (Wurtzit-BN). Darüber hinaus kann Bornitrid auch eine amorphe Struktur haben. Im wesentlichen unerheblich für die vorliegende Erfindung ist es, in welcher der natürlich vorkommenden Modifikationen das Borntrid vorliegt. Besonders bevorzugt ist amorphes Bornitrid.

Vorteilhaft im Sinne der vorliegenden Erfindung ist es, den mittleren Partikeldurchmesser der verwendeten Bornitridpartikel kleiner als 25 µm, besonders vorteilhaft kleiner als 7 µm zu wählen.

Vorteilhaft im Sinne der vorliegenden Erfindung sind beispielsweise die im folgenden aufgelisteten Bornitridtypen:

| **Handelsname** | **erhältlich bei** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bomitrid BNP | Wacker-Chemie |

Ebenfalls vorteilhaft im Sinne der vorliegenden Erfindung sind Bornitridpartikel, die oberflächlich wasserabweisend behandelt ("gecoatet") sind, wobei ein amphiphiler Charakter gebildet werden bzw. erhalten bleiben kann.

Eine vorteilhafte Beschichtung der Bornitridpartikel besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft sind beispielsweise die bei der Firma Carborundum unter der Handelsbezeichnung Très BN^{®} UHP 1106 erhältlichen, mit Dimethicone behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN^{®} UHP 1107 erhältlichen.

Die Gesamtmenge an einem oder mehreren Bornitriden in den fertigen kosmetischen oder dermatologischen Zubereitungen wird vorteilhaft aus dem Bereich von 0,1 bis 20,0 Gew.-%, bevorzugt 0,5 bis 10,0 Gew.-% gewählt, bezogen auf das Gesamtgewicht der Zubereitungen.

### Emulgatoren

Vorteilhaft im Sinne der vorliegenden Erfindung werden der oder die Emulgatoren gewählt aus der Gruppe der Glycerinester von α-Hydroxycarbonsäuren und gesättigten Fettsäuren, besonders vorteilhaft aus der Gruppe der Glycerinester von α-Hydroxycarbonsäuren, bei denen der α-Hydroxycarbonsäurerest einen Milchsäurerest 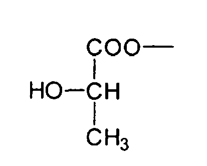 oder einen Citronensäurerest 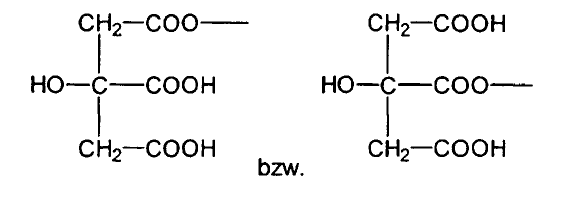 darstellt.

Vorteilhaft ist auch, den Fettsäurerest so zu wählen, daß R₁ einen C₁₃-C₁₉-Alkylrest darstellt.

Solche Citronensäureester sind beispielsweise erhältlich unter der Produktbezeichnung IMWITOR 370" der Gesellschaft Hüls AG.

Ferner vorteilhaft im Sinne der vorliegenden Erfindung sind Mischungen aus
(a) einer oder mehreren grenzflächenaktiven Substanzen A, gewählt aus der Gruppe der Glucosederivate, welche sich durch die Strukturformel 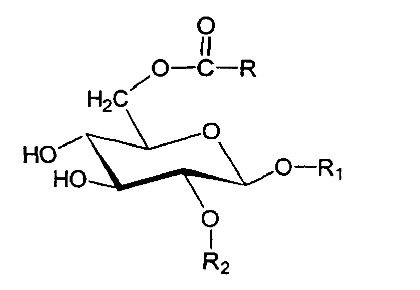 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, und
(b) einer oder mehreren grenzflächenaktiven Substanzen B, gewählt aus der Gruppe der Substanzen der allgemeinen Strukturformel 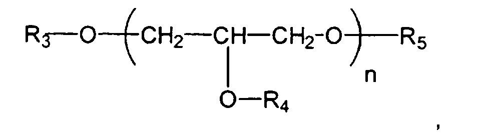 wobei R₃, R₄ und R₅ unabhängig voneinander gewählt werden aus der Gruppe, welche umfaßt: H, verzweigte und/oder unverzweigte, gesättigte und/oder ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

Besonders vorteilhaft wird oder werden die grenzflächenaktiven Substanzen A aus der Gruppe Methylglucosemonostearat (Formel wie nachstehend) 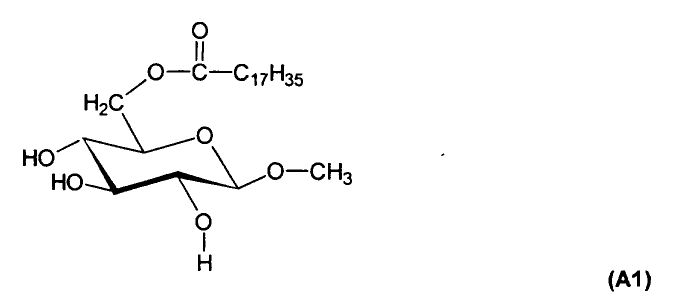 Methylglucosedistearat (Formel wie nachstehend) 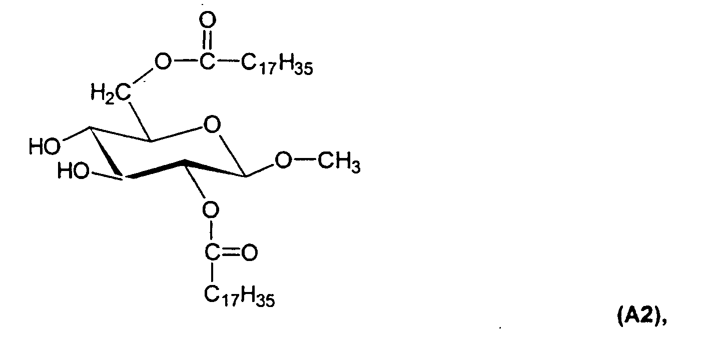 und beliebigen Gemischen daraus, beispielsweise ungefähr äquimolaren Mischungen daraus gewählt, welche auch als Methylglucosesesquistearat bezeichnet werden. Solches Methylglucosesesquistearat ist im Handel beispielsweise unter der Warenbezeichnung Tego® Care PS von der Gesellschaft Th. Goldschmidt KG erhältlich.

Besonders vorteilhaft wird oder werden die grenzflächenaktiven Substanzen B gewählt aus der Gruppe der Verbindungen, bei welcher n den Wert 3 annimmt und R₃, R₄ und R₅ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen:

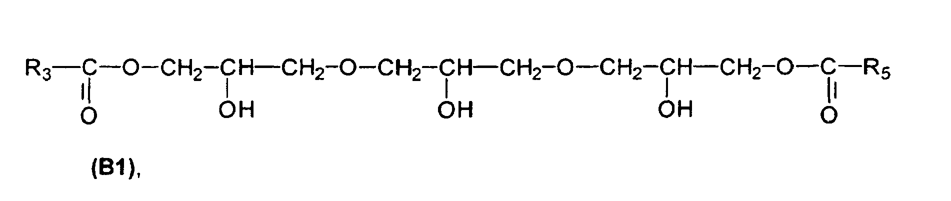

Als erfindungsgemäß bevorzugte Emulgatorkombination hat sich ein ungefähr äquimolekulares Gemisch aus den Verbindungen A2 und B1, wobei in B1 die Reste R₃ und R₅ vorzugsweise beide einen Stearatrest bezeichnen, herausgestellt. Solche Emulgatorkombinationen sind als Polyglyceryl(3)-Methylglucosedistearat" (PGMS) unter der Warenbezeichnung Tego Care® 450 von der Gesellschaft Th. Goldschmidt KG erhältlich.

Erfindungsgemäß vorteilhafte Emulgatoren sind ferner verzweigte und/oder unverzweigte Fettsäuren mit einer Kettenlänge von 14 bis 20 Kohlenstoffatomen, beispielsweise Stearinsäure und/oder Isostearinsäure.

Vorteilhaft im Sinne der vorliegenden Erfindung werden der oder die Emulgatoren ferner gewählt aus der Gruppe der Glycerinmono- und/oder Glycerindiester der Stearinsäure. Solche Emulgatoren sind beispielsweise als "Glycerl Stearate SE" bzw. "Glycerl Stearate" unter den Handelsbezeichnungen Tegin® bzw. Tegin® M von der Th. Goldschmidt KG und unter der Handelsbezeichnung Cutina GMS bei der Henkel KGaA erhältlich.

Erfindungsgemäß vorteilhaft sind ferner Sorbitanfettsäureester, welche Mischungen partieller Fettsäureester des Sorbit und seiner Anhydride (Sorbitan und Sorbid) mit Laurin-, Öl-, Palmitin- und Stearinsäure darstellen. Insbesondere vorteilhaft sind Sorbitandiisostearat (CAS-Nr.: 68238-87-9), Sorbitandioleat (CAS-Nr.: 29116-98-1), Sorbitanisostearat (CAS-Nr.:719020-10-7), Sorbitanlaurat (CAS-Nr.: 1338-39-2), Sorbitanoleat (CAS-Nr.: 1338-43-8), Sorbitanpalmitat (CAS-Nr.: 26266-57-9), Sorbitansesquistearat (CAS-Nr.: 71812-38-9), Sorbitansesquioleat (CAS-Nr.: 8007-43-0), Sorbitansesquistearate (CAS-Nr.: 51938-44-4), Sorbitanstearate (CAS-Nr.: 1338-41-6), Sorbitantristearate (CAS-Nr.: 54392-27-7 bzw. 26658-19-5) und/oder Sorbitantrioleate (CAS-Nr.: 26266-58-0).

### Ölphase

Die Ölphase der erfindungsgemäßen O/W-Emulsionen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, ver-zweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase der erfindungsgemäßen O/W-Emulsionen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Ferner kann die Ölphase der erfindungsgemäßen O/W-Emulsionen, wenngleich nicht zwingend, ebenfalls vorteilhaft auch unpolare Öle enthalten, beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

### Kosmetische oder dermatologische Hilfs- und Zusatzstoffe

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin; Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch weitere Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,-Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen O/W-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen enthalten, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-B-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere 5-Isopropyldibenzoylmethan (CAS-Nr. 63250-25-9), welches von Merck unter der Marke Eusolex^{®} 8020 verkauft wird, und/oder das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure: 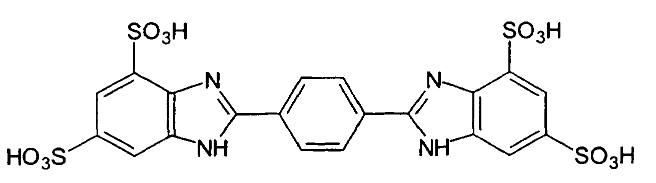 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz, sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

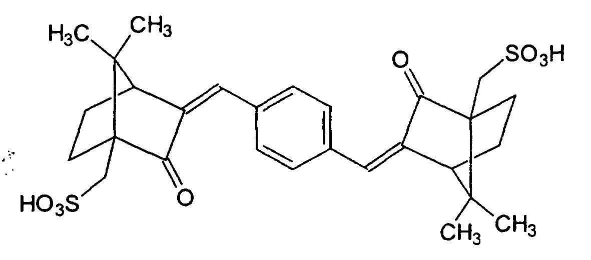

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter sind beispielsweise Bis-Resorcinyltriazinderivate mit der folgenden Struktur: 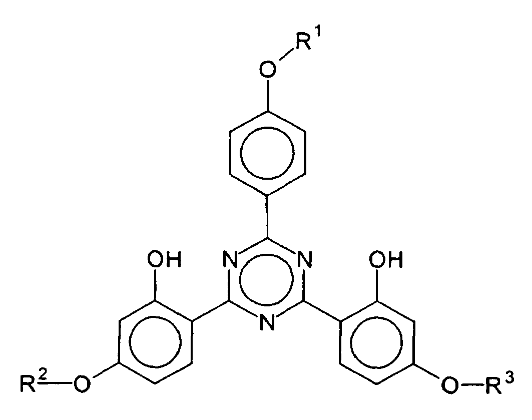 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyll-6-(4-methoxyphenyl)-1,3,5-triazin und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin, welches von der BASF Aktiengesellschaft unter der Warenbezeichnung UVINUL® T 150 vertrieben wird.

Auch andere UV-Filtersubstanzen beschrieben, welche das Strukturmotiv 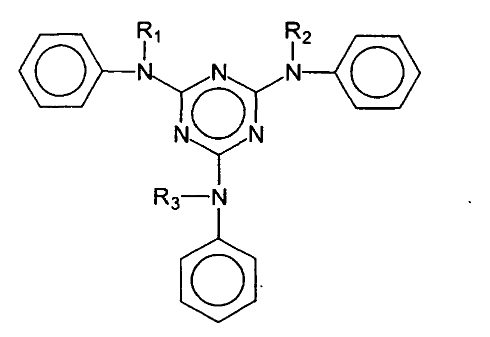 aufweisen sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 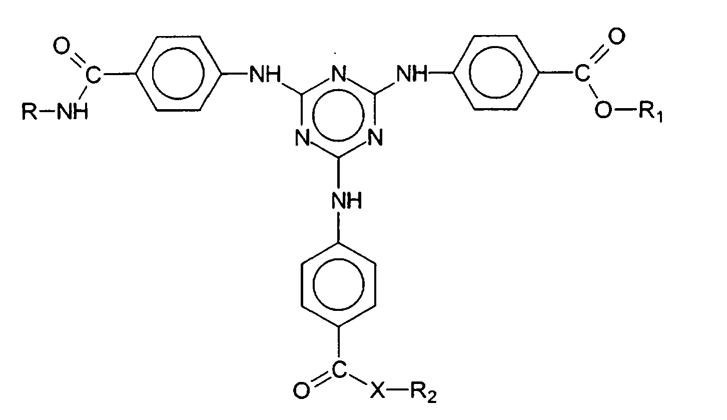 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 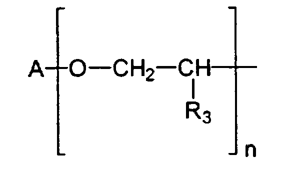 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 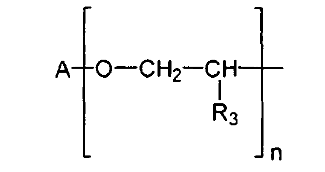 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt, wenn X ein Sauerstoffatom darstellt.

Ein Beispiel für solche unsymmetrisch substituierte s-Triazine stellt das Dioctylbutylamidotriazon dar.

Auch andere UV-Filtersubstanzen, deren Einarbeitung in kosmetische oder dermatologische Lichtschutzformulierungen bisher Probleme aufwies, sind bekannt. So werden in der Europäischen Offenlegungsschrift 775 698 Bis-Resorcinyltriazinderivate beschrieben, deren chemische Struktur durch die generische Formel 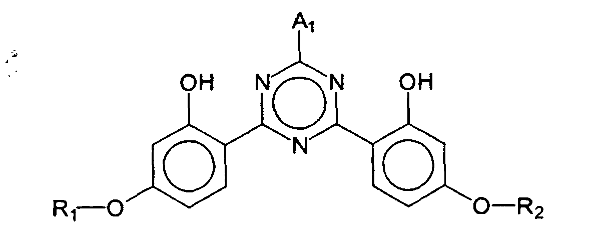 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propytoxy)-2-hydroxypropyloxy)-2-hydroxy]-phenyl}-6-[4-(2-ethyl-carboxyl)-phenylamino]-1,3,5-triazin, das 2,4-Bis-{[4-{2-ethyl-hexyloxy}-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy]-phenyl}-6-(4-methoxyphenyl)-1,3,5-triazin.

Ein weiterer vorteilhafter UV-Filter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 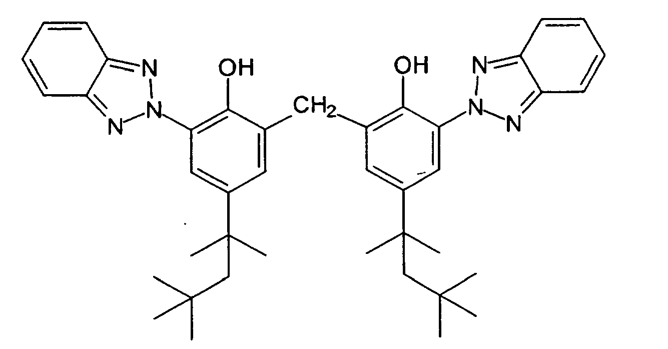 gekennzeichnet ist.

Vorteilhaft im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 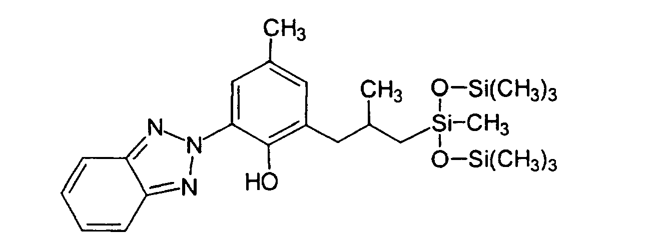 gekennzeichnet ist.

Die UV-B-Filter können öllöslich oder wasserlöslich sein. Vorteilhafte öllösliche UV-B-Filtersubstanzen sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

### Vorteilhafte wasserlösliche UV-B-Filtersubstanzen sind z.B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

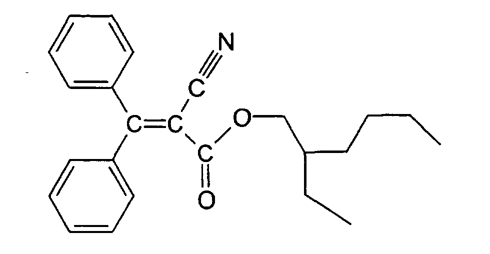

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A-und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

| | **O/W 1** | **O/W 2** | **O/W 3** | **O/W 4** | **O/W 5** |
|---|---|---|---|---|---|
| Stearinsäure | 1,5 | 1,5 | | | |
| Glycerinmonostearat | 3 | 3 | 5 | | |
| Sorbitanstearat | | | | 5 | 3 |
| Polyglyceryl-3 Methylglucose Distearat | | | | | 1,5 |
| Cetearyl Alkohol | | 2 | 0,5 | 0,5 | |
| CapryliclCapric Triglycerid | 10 | 5 | 5 | | |
| Octyldodecanol | | | | 5 | 10 |
| Dicaprylylether | 5 | | | 2 | 5 |
| Dimethicon | 2 | | | 2 1 | |
| Hydriertes Polyisobuten | 2 | | | | |
| Macadamianußöl | | 5 | | 2 | 2 |
| Butylen Glycol Dicaprylat/Caprat | | 5 | 5 | 5 | 5 |
| C₁₂₋₁₅- Alkylbenzoat | | 5 | 5 | | |
| Vitamen E-Acetat | 0,5 | 0,5 | 0,5 | 0,5 | 0,5 |
| Octyltriazon | 2 | 4 | 5 | 3 | 4 |
| Dioctyl Butamido Triazon | | 1 | 2 | | |
| Methylbenzyliden Campher | 4 | 2 | | 4 | 4 |
| Butyl Methoxydibenzoyl Methan | 2 | 2 | 1 | 1,5 | 3 |
| Bornitrid | 1 | 1 | 5 | 2 | 2 |
| Konservierung | 0,5 | 0,5 | 0,5 | 0,5 | 0,5 |
| Glycerin | 3 | 5 | 5 | 5 | 10 |
| Xanthan Gummi | 0,3 | | 0,1 | | 0,2 |
| Pemulen TR1^{a} | | 0,1 | 0,1 | 0,3 | 0,1 |
| Phenylbenzimidazol Sulfonsäure | | | | 4 | 2 |
| Natronlauge 45% | 0,5 | 0,6 | | 1,4 | 1,2 |
| Wasser | ad 100 | ad 100 | ad 100 | ad 100 | ad 100 |

| | | | | | |
|---|---|---|---|---|---|
| ^{a} Copolymer aus C₁₀₋₃₀ Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit (CTFA-Bez.: Acrylates/C 10-30 Alkyl Acrylate Crosspolymer) | | | | | |

## Patentansprüche

1. Kosmetische oder dermatologische O/W-Emulsionen mit einem Gehalt an mindestens einem Bornitrid, wobei der mittlere Partikeldurchmesser der verwendeten Bornitridpartikel kleiner als 25 µm beträgt, mindestens einer UV-Filtersubstanz gewählt aus der Gruppe der Verbindungen der Triazinderivate, Benzotriazole, Phenylen-1,4-bis-(2-benzimidazyl)-3,3'-5,5'-tetrasulfonsäure und ihrer Salze, 1,4-di(2-oxo-10-Sulfo-3-bornylidenmethyl)-Benzol und dessen Salze, Salze der 2-Phenylbenzimidazol-5-sulfonsäure sowie die Sulfonsäure selbst sowie Sulfonsäure-Derivate des 3-Benzylidencamphers und deren Salze und einer Ölkomponente, deren Grenzflächenspannung gegen Wasser kleiner als 35 mN/m beträgt.

2. Verwendung von mindestens einem Bornitrid, wobei der mittlere Partikeldurchmesser der verwendeten Bornitridpartikel kleiner als 25 µm beträgt, zum Erzielen oder Erhöhen der Stabilität von O/W-Emulsionen welche mindestens eine UV-Filtersubstanz gewählt aus der Gruppe der Verbindungen der Triazinderivate, Benzotriazole, Phenylen-1,4-bis-(2-benzimidazyl)-3,3'-5,5'-tetrasulfonsäure und ihrer Salze, 1,4-di(2-oxo-10-Sulfo-3-bornylidenmethyl)-Benzol und dessen Salze, Salze der 2-Phenylbenzimidazol-5-sulfonsäure sowie die Sulfonsäure selbst sowie Sulfonsäure-Derivate des 3-Benzylidencamphers und deren Salze und eine Ölkomponente, deren Grenzflächenspannung gegen Wasser kleiner als 35 mN/m beträgt, enthalten.

3. Verwendung von mindestens einem Bornitrid zum Erzielen oder Erhöhen der Stabilität von O/W-Emulsionen in Gegenwart von Ölkomponenten, deren Grenzflächenspannung gegen Wasser kleiner als 35 mN/m beträgt, und polaren UV-Filtern gewählt aus der Gruppe der Verbindungen der Triazinderivate, Benzotriazole, Phenylen-1,4-bis-(2-benzimidazyl)-3,3'-5,5'-tetrasulfonsäure und ihrer Salze, 1,4-di(2-oxo-10-Suffo-3-bomylidenmethyl)-Benzol und dessen Salze, Salze der 2-Phenylbenzimidazol-5-sulfonsäure sowie die Sulfonsäure selbst sowie Sulfonsäure-Derivate des 3-Benzylidencamphers.

4. O/W-Emulsion nach Anspruch 1 oder Verwendung nach einem der Ansprüche 2 und 3, **dadurch gekennzeichnet, daß** die Emulsion weniger als 2 Gew.%, insbesondere weniger als 1 Gew.-%, ganz besonders weniger als 0,5 Gew.-% Ölkomponenten bezogen auf das Gesamtgewicht der Zubereitungen enthält, deren Grenzflächenspannung gegen Wasser größer als 35 mN/m beträgt.

5. O/W-Emulsion nach Anspruch 1 oder Verwendung nach einem der Ansprüche 2 und 3, **dadurch gekennzeichnet, daß** der mittlere Partikeldurchmesser der verwendeten Bornitridpartikel kleiner als 7 µm gewählt wird.

6. O/W-Emulsion nach Anspruch 1 oder Verwendung nach einem der Ansprüche 2 und 3, **dadurch gekennzeichnet, daß** die Gesamtmenge an einem oder mehreren Bornitriden in den fertigen kosmetischen oder dermatologischen Zubereitungen aus dem Bereich von 0,1 bis 20,0 Gew.%, bevorzugt 0,5 bis 10,0 Gew.-% gewählt wird, bezogen auf das Gesamtgewicht der Zubereitungen.

7. O/W-Emulsion nach Anspruch 1 oder Verwendung nach einem der Ansprüche 2 und 4, **dadurch gekennzeichnet, daß** der oder die Emulgatoren gewählt werden aus Gruppe der Glycerinester von α-Hydroxycarbonsäuren und gesättigten Fettsäuren, Methylglucosemonostearat, Methylglucosedistearat, Polyglyceryl(3)-Methylglucosedistearat, der verzweigten und/oder unverzweigten Fettsäuren mit einer Kettenlänge von 14 bis 20 Kohlenstoffatomen, Glycerinmono- und/oder Glycerindiester der Stearinsäure sowie Sorbitanfettsäureester.

8. O/W-Emulsion nach Anspruch 1 oder Verwendung nach einem der Ansprüche 2 und 3, **dadurch gekennzeichnet, daß** mindestens eine UV-Filtersubstanz gewählt wird aus der Gruppe der Triazinderivate, insbesondere aus der Gruppe
(1,3,5-Triazin-2,4,6-triyltriimino)-tris-benzoesäure-tris(2-ethylhexylester),
2,4-Bis-{[4-(2-Ethyl-hexyloxy)-2-hydroxy]-phenyl}-6-(4-methoxyphenyl)-1,3,5-triazin,
2,4-Bis-{[4-(3-sulfonato)-2-hydroxy-propyloxy)-2-hydroxy]-phenyl}-6-(4-methoxyphe nyl)-1,3,5-triazin Natriumsalz,
2,4-Bis-{[4-(3-(2-Propyloxy)-2-hydroxy-propyloxy)-2-hydroxy]-phenyl}-6-(4-meth oxyphenyl)-1,3,5-triazin,
2,4-Bis-{[4-(2-ethyl-hexyloxy)-2-hydroxy]-phenyl}-6-[4-(2-methoxyethyl-carboxyl)-phe nylamino]-1,3,5-triazin,
2,4-Bis-{[4-(3-(2-propyloxy)-2-hydroxy-propyloxy)-2-hydroxy]-phenyl}-6-[4-(2-ethylcarboxyl)-phenylamino]-1,3,5-triazin,
2,4-Bis-{[4-(2-ethyl-hexyloxy)-2-hydroxy]-phenyl}-6-(1-methyl-pyrrol-2-yl)-1,3,5-triazin,
2,4-Bis-{[4-tris(trimethylsiloxy-silylpropyloxy)-2-hydroxy]-phenyl}-6-(4-methoxyphenyl)-1,3,5-triazin,
2,4-Bis-{[4-(2''-methylpropenyloxy)-2-hydroxy]-phenyl}-6-(4-methoxyphenyl)-1,3,5-triazin,
2,4-Bis-{[4-(1',1',1',3',5',5',5'-Heptamethylsiloxy-2''-methyl-propyloxy)-2-hydroxy]-phenyl}-6-(4-methoxyphenyl)-1,3,5-triazin.

9. O/W-Emulsion nach Anspruch 1 oder Verwendung nach einem der Ansprüche 2 und 3, **dadurch gekennzeichnet, daß** mindestens eine UV-Filtersubstanz gewählt wird aus der Gruppe
Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz,
1,4-di(2-oxo-10-Sulfo-3-bornylidenmethyl)-Benzol und dessen Salze, besonders die entprechenden 10-Sulfato-verbindungen, insbesondere das entsprechende Natrium-, Kalium- oder Triethanolammonium-Salz),
Salze der 2-Phenylbenzimidazol-5-sulfonsäure, wie ihr Natrium-, Kalium- oder ihr Triethanolammonium-Salz, sowie die Sulfonsäure selbst sowie
Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

10. O/W-Emulsion nach Anspruch 1 oder Verwendung nach einem der Ansprüche 2 und 3, **dadurch gekennzeichnet, daß** mindestens eine UV-Filtersubstanz gewählt wird aus der Gruppe der Benzotriazole, insbesondere aus der Gruppe 2,2'-Methylen-bis-(6-(2H-benzotriazol-2-yl)-4-(1,1,3,3-tetramethylbutyl)-phenol) und 2-(2H-benzotriazol-2-yl)-4-methyl-6-[2-methyl-3-[1,3,3,3-tetramethyl-1-[(trimethylsilyl)oxy]disiloxanyl]propyl]-phenol.

11. O/W-Emulsion nach Anspruch 1 oder Verwendung nach einem der Ansprüche 2 und 3, **dadurch gekennzeichnet, daß** zusätzlich weitere kosmetische oder pharmazeutische Hilfs-, Zusatz- und/oder Wirkstoffe enthalten sind.

## Claims

1. Cosmetic or dermatological O/W emulsions with a content of at least one boron nitride, where the average particle diameter of the boron nitride particles used is less than 25 µm, at least one UV filter substance chosen from the group of compounds of the triazine derivatives, benzotriazoles, phenylene-1,4-bis(2-benzimidazyl)-3,3',5,5'-tetrasulphonic acid and its salts, 1,4-di(2-oxo-10-sulpho-3-bornylidenemethyl)benzene and salts thereof, salts of 2-phenylbenzimidazole-5-sulphonic acid, and the sulphonic acid itself and sulphonic acid derivatives of 3-benzylidenecamphor and salts thereof, and an oil component whose interfacial tension towards water is less than 35 mN/m.

2. Use of at least one boron nitride, where the average particle diameter of the boron nitride particles used is less than 25 µm, for achieving or increasing the stability of O/W emulsions which comprise at least one UV filter substance chosen from the group of compounds of the triazine derivatives, benzotriazoles, phenylene-1,4-bis(2-benzimidazyl)-3,3',5,5'-tetrasulphonic acid and its salts, 1,4-di(2-oxo-10-sulpho-3-bornylidenemethyl)benzene and salts thereof, salts of 2-phenylbenzimidazole-5-sulphonic acid, and the sulphonic acid itself and sulphonic acid derivatives of 3-benzylidenecamphor and salts thereof, and an oil component whose interfacial tension towards water is less than 35 mN/m.

3. Use of at least one boron nitride for achieving or increasing the stability of O/W emulsions in the presence of oil components whose interfacial tension towards water is less than 35 mN/m, and polar UV filters chosen from the group of compounds of the triazine derivatives, benzotriazoles, phenylene-1,4-bis(2-benzimidazyl)-3,3',5,5'-tetrasulphonic acid and its salts, 1,4-di(2-oxo-10-sulpho-3-bornylidenemethyl)benzene and salts thereof, salts of 2-phenylbenzimidazole-5-sulphonic acid, and the sulphonic acid itself, and sulphonic acid derivatives of 3-benzylidenecamphor.

4. O/W emulsion according to Claim 1 or use according to one of Claims 2 and 3, **characterized in that** the emulsion comprises less than 2% by weight, in particular less than 1% by weight, very particularly less than 0.5% by weight, of oil components, based on the total weight of the preparations, whose interfacial tension towards water is greater than 35 mN/m.

5. O/W emulsion according to Claim 1 or use according to one of Claims 2 and 3, **characterized in that** the average particle diameter of the boron nitride particles used is chosen to be less than 7 µm.

6. O/W emulsion according to Claim 1 or use according to one of Claims 2 and 3, **characterized in that** the total amount of one or more boron nitrides in the finished cosmetic or dermatological preparations is chosen from the range from 0.1 to 20.0% by weight, preferably 0.5 to 10.0% by weight, based on the total weight of the preparations.

7. O/W emulsion according to Claim 1 or use according to one of Claims 2 and 3, **characterized in that** the emulsifier or the emulsifiers is/are chosen from the group of glycerol esters of α-hydroxycarboxylic acids and saturated fatty acids, methylglucose monostearate, methylglucose distearate, polyglyceryl(3)-methylglucose distearate, branched and/or unbranched fatty acids having a chain length of from 14 to 20 carbon atoms, glycerol monoesters and/or glycerol diesters of stearic acid, and sorbitan fatty acid esters.

8. O/W emulsion according to Claim 1 or use according to one of Claims 2 and 3, **characterized in that** at least one UV filter substance is chosen from the group of triazine derivatives, in particular from the group
tris(2-ethylhexyl) (1,3,5-triazine-2,4,6-triyltriimino)trisbenzoate,
2,4-bis{[4-(2-ethylhexyloxy)-2-hydroxy]phenyl}-6-(4-methoxyphenyl)-1,3,5-triazine,
2,4-bis{[4-(3-sulphonato)-2-hydroxypropyloxy)-2-hydroxy]phenyl}-6-(4-methoxyphenyl)-1,3,5-triazine sodium salt,
2,4-bis{[4-(3-(2-propyloxy)-2-hydroxypropyloxy)-2-hydroxy]phenyl}-6-(4-methoxyphenyl)-1,3,5-triazine,
2,4-bis{[4-(2-ethylhexyloxy)-2-hydroxy]phenyl}-6-[4-(2-methoxyethylcarboxyl)phenylamino]-1,3,5-triazine,
2,4-bis{[4-(3-(2-propyloxy)-2-hydroxypropyloxy)-2-hydroxy]phenyl}-6-[4-(2-ethylcarboxyl)phenylamino]-1,3,5-triazine,
2,4-bis{[4-(2-ethylhexyloxy)-2-hydroxy]phenyl}-6-(1-methylpyrrol-2-yl)-1,3,5-triazine,
2,4-bis{[4-tris(trimethylsiloxysilylpropyloxy)-2-hydroxy]phenyl}-6-(4-methoxyphenyl)-1,3,5-triazine,
2,4-bis{[4-(2"-methylpropenyloxy)-2-hydroxy]-phenyl}-6-(4-methoxyphenyl)-1,3,5-triazine,
2,4-bis{[4-(1',1',1',3',5',5',5'-heptamethylsiloxy-2"-methylpropyloxy)-2-hydroxy]phenyl}-6-(4-methoxyphenyl)-1,3,5-triazine.

9. O/W emulsion according to Claim 1 or use according to one of Claims 2 and 3, **characterized in that** at least one UV filter substance is chosen from the group
phenylene-1,4-bis(2-benzimidazyl)-3,3'-5,5'-tetrasulphonic acid and its salts, particularly the corresponding sodium, potassium or triethanolammonium salts, in particular phenylene-1,4-bis(2-benzimidazyl)-3,3'-5,5'-tetrasulphonic acid bis-sodium salt,
1,4-di(2-oxo-10-sulpho-3-bornylidenemethyl)benzene and salts thereof, particularly the corresponding 10-sulphato compounds, in particular the corresponding sodium, potassium or triethanolammonium salt,
salts of 2-phenylbenzimidazole-5-sulphonic acid, such as its sodium, potassium or triethanolammonium salt, and the sulphonic acid itself, and sulphonic acid derivatives of 3-benzylidenecamphor, such as, for example, 4-(2-oxo-3-bornylidenemethyl)benzenesulphonic acid, 2-methyl-5-(2-oxo-3-bornylidenemethyl)sulphonic acid and salts thereof.

10. O/W emulsion according to Claim 1 or use according to one of Claims 2 and 3, **characterized in that** at least one UV filter substance is chosen from the group of benzotriazoles, in particular from the group
2,2'-methylenebis(6-(2H-benzotriazol-2-yl)-4-(1,1,3,3-tetramethylbutyl)phenol) and 2-(2H-benzotriazol-2-yl)-4-methyl-6-[2-methyl-3-[1,3,3,3-tetramethyl-1-[(trimethylsilyl)oxy]disiloxanyl]propyl]phenol.

11. O/W emulsion according to Claim 1 or use according to one of Claims 2 and 3, **characterized in that** further cosmetic or pharmaceutical auxiliaries, additives and/or active ingredients are additionally present.

## Revendications

1. Emulsions H/E cosmétiques ou dermatologiques ayant une teneur en au moins un nitrure de bore, le diamètre moyen des particules de nitrure de bore utilisées étant inférieur à 25 µm, en au moins un filtre UV choisi dans le groupe des composés consistant en les dérivés de triazine, les benzotriazoles, l'acide phénylène-1,4-bis(2-benzimidazyl)-3,3'-5,5'-tétrasulfonique et ses sels, le 1,4-di(2-oxo-10-sulfo-3-bornylidèneméthyl)benzène et ses sels, les sels de l'acide 2-phénylbenzimidazole-5-sulfonique ainsi que l'acide sulfonique lui-même, ainsi que les dérivés sulfoniques du 3-benzylidènecamphre et leurs sels, et en un composant huileux dont la tension superficielle vis-à-vis de l'eau est inférieure à 35 mN/m.

2. Utilisation d'au moins un nitrure de bore, le diamètre moyen des particules de nitrure de bore utilisées étant inférieur à 25 µm, pour l'obtention ou l'augmentation de la stabilité d'émulsions H/E qui contiennent au moins un filtre UV choisi dans le groupe des composés consistant en les dérivés de triazine, les benzotriazoles, l'acide phénylène-1,4-bis(2-benzimidazyl)-3,3'-5,5'-tétrasulfonique et ses sels, 1,4-di(2-oxo-10-sulfo-3-bornylidèneméthyl)benzène et ses sels, les sels de l'acide 2-phénylbenzimidazole-5-sulfonique ainsi que l'acide sulfonique lui-même, ainsi que les dérivés sulfoniques du 3-benzylidènecamphre et leurs sels, et en un composant huileux dont la tension superficielle vis-à-vis de l'eau est inférieure à 35 mN/m.

3. Utilisation d'au moins un nitrure de bore pour l'obtention ou l'augmentation de la stabilité d'émulsions H/E en présence de composants huileux dont la tension superficielle vis-à-vis de l'eau est inférieure à 35 mN/m, et de filtres UV polaires choisis dans le groupe des composés consistant en les dérivés de triazine, les benzotriazoles, l'acide phénylène-1,4-bis(2-benzimidazyl)-3,3'-5,5'-tétrasulfonique et ses sels, le 1,4-di(2-oxo-10-sulfo-3-bornylidène-méthyl)benzène et ses sels, les sels de l'acide 2-phénylbenzimidazole-5-sulfonique ainsi que l'acide sulfonique lui-même, ainsi que les dérivés sulfoniques du 3-benzylidènecamphre.

4. Emulsion H/E selon la revendication 1 ou utilisation selon l'une quelconque des revendications 2 et 3, **caractérisée en ce que** l'émulsion contient moins de 2 % en poids, en particulier moins de 1 % en poids, tout particulièrement moins de 0,5 % en poids de composants huileux, par rapport au poids total des préparations, dont la tension superficielle vis-à-vis de l'eau est supérieure à 35 mN/m.

5. Emulsion H/E selon la revendication 1 ou utilisation selon l'une quelconque des revendications 2 et 3, **caractérisée en ce que** le diamètre moyen des particules de nitrure de bore utilisées est choisi inférieur à 7 µm.

6. Emulsion H/E selon la revendication 1 ou utilisation selon l'une quelconque des revendications 2 et 3, **caractérisée en ce que** la quantité totale d'un ou plusieurs nitrures de bore dans les préparations cosmétiques ou dermatologiques finales est choisie dans la plage allant de 0,1 à 20,0 % en poids, de préférence de 0,5 à 10,0 % en poids, par rapport au poids total des préparations.

7. Emulsion H/E selon la revendication 1 ou utilisation selon l'une quelconque des revendications 2 et 3, **caractérisée en ce que** l'émulsifiant ou les émulsifiants sont choisis dans le groupe constitué par les esters de glycérol et d'acides α-hydroxycarboxyliques et d'acides gras saturés, le monostéarate de méthylglucose, le distéarate de méthylglucose, le polyglycéryl(3)-distéarate de méthylglucose, les acides gras ramifiés et/ou non ramifiés ayant une longueur de chaîne de 14 à 20 atomes de carbone, le monoester de glycérol de l'acide stéarique et/ou le diester de glycérol de l'acide stéarique, ainsi que les esters d'acides gras et de sorbitanne.

8. Emulsion H/E selon la revendication 1 ou utilisation selon l'une quelconque des revendications 2 et 3, **caractérisée en ce qu'**au moins un filtre UV est choisi dans le groupe des dérivés de triazine, en particulier dans le groupe constitué par
le (1,3,5-triazine-2,4,6-triyltriimino)-tris-benzoate de tris(2-éthylhexyle),
la 2,4-bis{[4-(2-éthyl-hexyloxy)-2-hydroxy]-phényl}-6-(4-méthoxyphényl)-1,3,5-triazine,
le sel de sodium de 2,4-bis{[4-(3-sulfonato-2-hydroxy-propyloxy)-2-hydroxy]-phényl}-6-(4-méthoxyphényl)-1,3,5-triazine,
la 2,4-bis{[4-(3-(2-propyloxy)-2-hydroxy-propyloxy)-2-hydroxy]-phényl}-6-(4-méthoxyphényl)-1,3,5-triazine,
la 2,4-bis{[4-(2-éthyl-hexyloxy)-2-hydroxy]-phényl}-6-[4-(2-méthoxyéthylcarboxy)-phénylàmino]-1,3,5-triazine,
la 2,4-bis{[4-3-(2-propyloxy)-2-hydroxy-propyloxy)-2-hydroxy]-phényl}-6-[4-(2-éthylcarboxy)-phénylamino]-1,3,5-triazine,
la 2,4-bis{[4-(2-éthyl-hexyloxy)-2-hydroxy]-phényl}-6-(1-méthyl-pyrrol-2-yl)-1,3,5-triazine,
la 2,4-bis{[4-tris(triméthylsiloxy-silyl-propyloxy)-2-hydroxy]-phényl}-6-(4-méthoxyphényl)-1,3,5-triazine,
la 2,4-bis{[4-(2"-méthylpropényloxy)-2-hydroxy]-phényl}-6-(4-méthoxyphényl)-1,3,5-triazine,
la 2,4-bis{[4-(1',1',1',3',5',5',5'-heptaméthylsiloxy-2"-méthyl-propyloxy)-2-hydroxy]phényl}-6-(4-méthoxyphényl)-1,3,5-triazine.

9. Emulsion H/E selon la revendication 1 ou utilisation selon l'une quelconque des revendications 2 et 3, **caractérisée en ce qu'**au moins un filtre UV est choisi dans le groupe constitué par
l'acide phénylène-1,4-bis(2-benzimidazyl)-3,3'-5,5'-tétrasulfonique et ses sels, en particulier les sels de sodium, potassium ou triéthanolammonium correspondants, notamment le sel disodique d'acide phénylène-1,4-bis(2-benzimidazyl)-3,3'-5,5'-tétrasulfonique,
le 1,4-di(2-oxo-10-sulfo-3-bornylidèneméthyl)-benzène et ses sels, en particulier les composés 10-sulfato correspondants, notamment le sel de sodium, potassium ou triéthanolammonium correspondant,
les sels de l'acide 2-phénylbenzimidazole-5-sulfonique, tels que son sel de sodium, potassium ou son sel de triéthanolammonium, ainsi que l'acide sulfonique lui-même, ainsi que
les dérivés sulfoniques du 3-benzylidènecamphre, comme par exemple l'acide 4-(2-oxo-3-bornylidèneméthyl)benzènesulfonique, l'acide 2-méthyl-5-(2-oxo-3-bornylidèneméthyl)sulfonique et leurs sels.

10. Emulsion H/E selon la revendication 1 ou utilisation selon l'une quelconque des revendications 2 et 3, **caractérisée en ce que** qu'au moins un filtre UV est choisi dans le groupe des benzotriazoles, en particulier dans le groupe constitué par
le 2,2'-méthylène-bis(6-(2H-benzotriazol-2-yl)-4-(1,1,3,3-tétraméthylbutyl)-phénol) et
le 2-(2H-benzotriazol-2-yl)-4-méthyl-6-[2-méthyl-3-[1,3,3,3-tétraméthyl-1-[(triméthylsilyl)oxy]-disiloxanyl]propyl]phénol.

11. Emulsion H/E selon la revendication 1 ou utilisation selon l'une quelconque des revendications 2 et 3, **caractérisée en ce qu'**elle contient en outre d'autres adjuvants, additifs et/ou substances actives.
